Europäisches Patentamt

European Patent Office

Office européen des brevets

(11) Publication number: **0 055 109**
**A2**

## (12) EUROPEAN PATENT APPLICATION

(21) Application number: 81305981.3

(22) Date of filing: 21.12.81

(51) Int. Cl.³: **A 61 K 7/16**

(30) Priority: 24.12.80 US 220202

(43) Date of publication of application: 30.06.82
Bulletin 82/26

(84) Designated Contracting States: **AT BE CH DE FR GB IT LI LU NL SE**

(71) Applicant: WARNER-LAMBERT COMPANY, 201 Tabor Road, Morris Plains New Jersey 07950 (US)

(72) Inventor: Dills, Steven Searight, 1100 Parsippany Boulevard Apt. 213, Parsippany New Jersey 07054 (US)

(74) Representative: Jones, Michael Raymond et al, Haseltine Lake & Co. 28 Southampton Buildings Chancery Lane, London WC2A 1AT (GB)

(54) Anticaries composition.

(57) A composition for reducing the incidence and severity of human dental caries is disclosed. The composition comprises fluoride ion (such as that of sodium fluoride, lithium fluoride, hydrogen fluoride, stannous fluoride, rubidium fluoride, zirconium tetrafluoride or sodium fluorophosphate) and a nonmetabolizable analogue of a cariogenic carbohydrate (such as a nonmetabolizable analogue of glucose, fructose, maltose, lactose or sucrose), in association with a pharmaceutically acceptable carrier. The nonmetabolizable carrier is preferably 2-deoxy-D-glucose, glucosamine or a pharmaceutically acceptable salt of glucosamine. The composition inhibits the carbohydrate induced production of acid by human dental plaque bacteria.

ACTORUM AG

-1-

## ANTICARIES COMPOSITION

This invention relates to an anticaries composition for inhibiting the carbohydrate induced production of acid by human dental plaque bacteria, thereby reducing the incidence or severity of human dental caries.

It is believed that certain acidogenic bacteria, in particular <u>Streptococcus</u> <u>mutans</u> and several <u>Lactobacillus</u> species, e.g., <u>L</u>. <u>acidophilus</u>, are the predominant cariogenic microorganisms present in human dental plaque. These microorganisms when presented with metabolizable carbohydrates, such as glucose, fructose, lactose, maltose and sucrose, produce acid metabolic end products, e.g., acetic acid, formic acid and lactic acid. These acids are believed to be the major causative agents of human dental caries. It would therefore be beneficial to provide a means whereby the carbohydrate induced acid production by human dental plaque bacteria would be inhibited, thereby reducing the incidence and severity of human dental caries.

According to the present invention, there is provided a composition for reducing the incidence or severity of human dental caries, the composition comprising fluoride ion and a nonmetabolizable analogue of a cariogenic carbohydrate, in association with a pharmaceutically acceptable carrier.

The present invention also provides, for use in reducing the incidence or severity of human dental caries, fluoride ion and a nonmetabolizable analogue of a cariogenic carbohydrate.

The term "cariogenic carbohydrate" (hereinafter, cariogen) used herein means any carbohydrate that is metabolized by human dental plaque bacteria to cause human dental caries. Examples of such cariogens are glucose, fructose, maltose, lactose, sucrose and the like.

The term "nonmetabolizable analogue of a cariogenic carbohydrate" (hereinafter, cariogen analogue) used herein means a structural analogue of a cariogen which does not. support detectable growth within 24 hours of acidogenic human plaque bacteria when cultured under standard laboratory conditions.

The inhibitory effect of fluoride ion on the carogen induced acid production of Streptococcus mutans, one of the predominant acidogenic bacteria present in human dental plaque, has been studied: I.R. Hamilton and D.C. Ellwood, Infection and Immunity, 19, 434 (1978). As one might predict, the amount of inhibition observed varies with the fluoride ion concentration. Unfortunately, the amount of fluoride ion necessary for substantially complete inhibition would result in deleterious toxic effects when administered to a human host. Thus, the use of fluoride ion alone is not a completely effective means for eliminating human dental caries.

It is also known that carbohydrate induced acid production by Streptococcus mutans is inhibited by certain cariogen analogues, e.g., 2-deoxy-D-glucose (see C.F. Schachtele and W.S. Leung, J. Dent. Res., 54 433 (1975)). However, it has been proposed that the in vivo use of certain of these cariogen analogues is not practical since they interfere with mammalian metabolism (K.R. Roberts and M.L. Hayes, Scand. J. Dent. Res., 88, 201 (1980)). The use of cariogen analogues, in vivo, at the lowest possible concentration is therefore preferred.

It has now been discovered that the use of a combination of fluoride ion and a cariogen analogue, the fluoride constituent being present at a concentration which is noninjurious to a human hose, will effectively inhibit the cariogen induced acid production of human dental plaque bacteria thereby reducing the incidence of and severity of human dental caries. It has further been discovered that when utilized in combination, each constituent may be present at a concentration where neither would be a clinically efficacious anti-caries agent if utilized alone. Incidentally, while the toxicity of cariogen analogues such as 2-deoxy-D-glucose has not been established, the in vivo use of certain analogues has been reported. Thus, H.A. Glough and R.L. Giuntoli, J.A.M.A., 241, 2798 (1979), have reported the topical use of 2-deoxy-D-glucose with no deleterious effects observed.

For a better understanding of the invention, reference will now be made, by way of example, to the accompanying drawings, which are graphs showing the production of acid (measured on the ordinate as pH) relative to time (measured on the abscissa in minutes). In the drawings:

Figure I relates to the effect of 2-deoxy-D-glucose and sodium fluoride on the production of acid by Streptococcus mutans g 6715 from sucrose, wherein:

line 1 relates to the use of water as a control,

line 2 relates to the use of 5 mM of sucrose,

line 3 relates to the use of 5 mM of sucrose plus 0.5 mM of sodium fluoride,

line 4 relates to the use of 5 mM of sucrose plus 5 mM of 2-deoxy-D-glucose, and

line 5 relates to the use of 5 mM of sucrose plus 5 mM of 2-deoxy-D-glucose plus 0.5 mM of sodium fluoride;

0055109

Figure II relates to the effect of 2-deoxy-D-glucose and sodium fluoride on the production of acid by Lactobacillus acidophilus ATCC 4356 from sucrose, wherein:

line 1 relates to the use of water as a control,

line 2 relates to the use of 10 mM of sucrose,

line 3 relates to the use of 10 mM of sucrose plus 1 mM of sodium fluoride,

line 4 relates to the use of 10 mM of sucrose plus 20 mM of 2-deoxy-D-glucose, and

line 5 relates to the use of 10 mM of sucrose plus 20 mM of 2-deoxy-D-glucose plus 1 mM of sodium fluoride;

Figure III relates to the effect of 2-deoxy-D-glucose and sodium fluoride on the production of acid by three day old human dental plaque from sucrose wherein:

line 1 relates to the use of water as a control,

line 2 relates to the use of 10 mM of sucrose,

line 3 relates to the use of 10 m M of sucrose plus 10 mM of 2-deoxy-D-glucose,

line 4 relates to the use of 10 mM of sucrose plus 10 mM of sodium fluoride, and

line 5 relates to the use of 10 mM of sucrose plus 10 mM of 2-deoxy-D-glucose plus 10 mM of sodium fluoride;

Figure IV relates to the effect of D-glucosamine hydrochloride and sodium fluoride on the production of acid by Streptococcus mutans g 6715 from sucrose, wherein:

line 1 relates to the use of 10 mM of sucrose,

line 2 relates to the use of 10 mM of sucrose plus 5 mM of sodium fluoride,

line 3 relates to the use of 10 mM of sucrose plus 10 mM of glucosamine hydrochloride, and

line 4 relates to the use of 10 mM of sucrose plus 10 mM of glucosamine hydrochloride plus 5 mM of sodium fluoride.

To obtain the results shown in the Figures, cell

suspensions of <u>Streptococcus</u> <u>mutans</u> and <u>Lactobacillus</u> <u>acidophilus</u> were prepared by harvesting log phase cell cultures, washing them with 1 mm potassium phosphate buffer, pH 7.0, then resuspending them to 100 mg cell dry weight per ml. Three-day human plaque was harvested from 5 individuals pooled and suspended in 1 mm potassium phosphate buffer, pH 7.0., by mild sonication. Measurements of acid production following cariogen addition (sucrose, fructose, glucose, etc.) were made by monitoring hydrogen ion concentrations over time with a Radiometer RTS 822 Titration Recording System. Assay conditions were as follows, temperature, $37^{O}C$ with constant stirring, two ml reaction volume containing 1 mM potassium phosphate, 0.1 to 10 mg cell dry weight. Cariogen and inhibitor additions as specified with the experiment.

In Figure I, acid production by <u>Streptococcus</u> <u>mutans</u> was monitored by pH measurements of the cell suspension medium for 30 minutes following addition of the specific cariogen, sucrose. The addition of sucrose (5 mM) elicited an immediate pH drop which leveled off to a slow pH decline after approximately 15 minutes. The pH at the end of the incubation period was ca. 4.3. Simultaneous addition of 0.5 mM fluoride ion as sodium fluoride with 5 mM sucrose, did not significantly affect the magnitude of the pH decline. Similarly, addition of 5 mM 2-deoxy-D-glucose (a cariogen analogue, C.F. Schachtele <u>supra</u>) with 5 mM sucrose caused no reduction in either the rate or magnitude of the pH decline when compared to 5 mM

sucrose alone. However, the combined addition of 0.5 mM sodium fluoride and 5 mM 2-deoxy-D-glucose reduced the level of acid production from 5 mM sucrose to levels produced by cells to which no cariogen had been added ($H_2O$ control). In these experiments it was observed that the combined addition of a cariogen analogue (2-deoxy-D-glucose) and fluoride ion (sodium fluoride) resulted in a significantly greater reduction in the acidogenic capabilities of Streptococcus mutans than did the addition of either component alone.

The combined inhibitory effects of the cariogen analogue, 2-deoxy-D-glucose and fluoride ion (sodium fluoride) on acid production induced by the cariogen, sucrose, were also observed with suspensions of Lactobacillus acidophilus (figure II) and 3-day human plaque (figure III). L. acidophilus and 3-day human plaque appeared more sensitive to fluoride ion inhibition than S. mutans. However, the combined addition of 2-deoxy-D-glucose with fluoride ion caused significantly greater inhibition than when either constituent of the combination was used alone.

Figure IV gives the results of an experiment with Streptococcus mutans showing the combined effects of cariogen analogue D-glucosamine-HCl and fluoride ion on acid production from sucrose by this organism. In the control run, with no inhibitors, the pH rapidly declined following sucrose addition to pH 4.5 within 5 minutes. The rate of decline then leveled off finally reaching a pH of ca. 3.8 at 30 minutes. The individual use of either 5 mM flouride ion or 10 mM glucosamine-HCl significantly reduced the magnitude of the sucrose induced pH decline. When these two materials were used in combination, the magnitude of the pH decline was significantly less than when either was used alone. No pH decline was observed without sucrose addition.

The fluoride ion used in the practice of the invention can be provided from any source. Thus, for example sodium fluoride, hydrogen fluoride, lithium fluoride, stannous fluoride, rubidium fluoride, zirconium tetrafluoride and sodium fluorophosphate may be used. Mixtures of these and similar compositions may also be utilized as the source of the fluoride ion. Stannous fluoride and sodium fluoride are the preferred sources of fluoride ion, sodium fluoride is the most preferred source.

Many cariogen analogues are known and may be used in the practice of the invention. These include, for example, aminated, alkylated, alkoxylated, halogenated, phosphorylated, sulfated, esterified, deoxy and sulfur derivatives of the respective cariogens, and may also include polyderivatized cariogen analogues. Specific examples of such cariogen analogues are 5-thio-D-glucose, 2-deoxy-D-glucose, 2-chloro-2-deoxy-D-glucose, 2-fluoro-2-deoxy-D-glucose, 2-0-methyl-D-glucose, 3-0-methyl-D-glucose, 4-0-methyl-D-glucose, 1-0-methyl-D-glucose, glucose-2-phosphate, 1,5-anhydroglucitol, D-glucosamine, N-acetyl-D-glucosamine, 6-chloro-6-deoxy-D-fructose, $\alpha$-D-2-deoxyglucopyranosyl-$\beta$-D-fructofuranose, $\alpha$-D-2-phospho-2-deoxyglucopyranosyl-$\beta$-D-fructofuranose and the like. This list is exemplary only, and other such analogues will be familiar to those skilled in the art. In addition, mixtures of the cariogen analogues may also be used. The free base forms of cariogen analogues which are basic in nature, e.g. glucosamine, are considered equivalent to their pharmaceutically acceptable salt forms for purposes of the invention. Examples of pharmaceutically acceptable acids useful for preparing such salts are hydrochloric, hydrofluoric, sulfuric, phosphoric, acetic, fumaric, citric, maleic, benzoic, malic and the like. The cariogen analogues preferred for purposes of the present invention are 2-deoxy-D-glucose and glucosamine.

Examples of cariogens contemplated by the invention are sucrose, glucose, fructose, maltose, lactose, mannose and the like; as well as mixtures thereof.

The pharmaceutically acceptable carriers contemplated by the invention are well known to those skilled in the art and are exemplified by any substantially non-toxic material or mixture of materials which can be utilized to place the fluoride ion and the cariogen analogue combination in contact with human dental plaque, and thereby in contact with human dental plaque bacteria which are exemplified by species such as Streptococcus mutans, Lactobacillus acidophilus and the like. This contact is intended to take place in the mouth. Thus, simply providing the combination of fluoride ion and cariogen analogue in solution or suspension for use as a mouth rinse is sufficient. The addition of colorants, detergents, flavors, sweeteners, stabilizers and other adjuvants to such a solution or suspension is optional. In addition, the combination may be added to any standard preparation normally used in the mouth; such as mouth rinses, mouth washes, mouth deoderizers, gargles, toothpastes, tooth powders and the like.

The combination of fluoride ion and cariogen analogue is particularly suited to be added to cariogen containing food products which remain in the mouth for an extended period of time. Carbohydrate sweetened confections such as candies or chewing gums are intended to remain in the mouth for extended periods. These confections may derive all or a portion of their sweetness from metabolizable carbohydrates such as fructose and/or sucrose which will lead to prolonged acidic conditions. This is due to the organic acid metabolic end products which are produced by the human dental plaque bacteria. The prolonged acidic conditions of course, will lead to tooth demineralization. The presence of the combination with such confections in the mouth will effectively reduce acid production, thereby limiting the incidence and severity of human dental caries caused by the carbohydrates present in such confections.

The individual components of the combination, i.e., fluoride ion and the cariogen analogue, may be present in a wide range of concentrations which would not be deleterious to a human receiving said combination. Thus, the fluoride ion component may be present in concentrations ranging from about 1 mmolar to about 50 mmolar and the cariogen analogue component may be present in concentrations ranging from about 10 mmolar to about 500 mmolar. The preferred concentration ranges for these components are as follows: fluoride ion, 25 mmolar to 50 mmolar; cariogen analogue, 10 mmolar to 100 mmolar. The most preferred composition contemplated by the invention comprises sodium fluoride at a concentration from 40 mmolar to 50 mmolar and 2-deoxy-D-glucose at a concentration from 25 mmolar to 50 mmolar.

The following are examples of oral care product formulations which may be used to place the combination of fluoride ion and the cariogen analogue in contact with human dental plaque. It will be understood that these formulations may be produced using standard methods, standard machinery and standard manufacturing techniques:

### Dentifrice

| | | |
|---|---|---|
| Dicalcium phosphate | 45 | % |
| Glycerine | 8 | % |
| Detergent | 10 | % |
| Whitener | 1 | % |
| Flavor including sweetener | 1 | % |
| Thickener and emulsifiers | 17 | % |
| Sodium Fluoride | 1.5 | % |
| 2-deoxy-D-glucose | .8 | % |
| Water | q.s. | |
| | 100 | % |

0055109

## Oral Rinse

| | | |
|---|---|---|
| Flavor | 1 | % |
| Alcohol | 20 | % |
| Sweeteners | 1 | % |
| Thickener | .6 | % |
| Coloring Agent | .01% | |
| Glycerine | 7 | % |
| Sodium Fluoride | .20% | |
| 2-deoxy-D-Glucose | .8 | % |
| Water | q.s. | |
| | 100 | % |

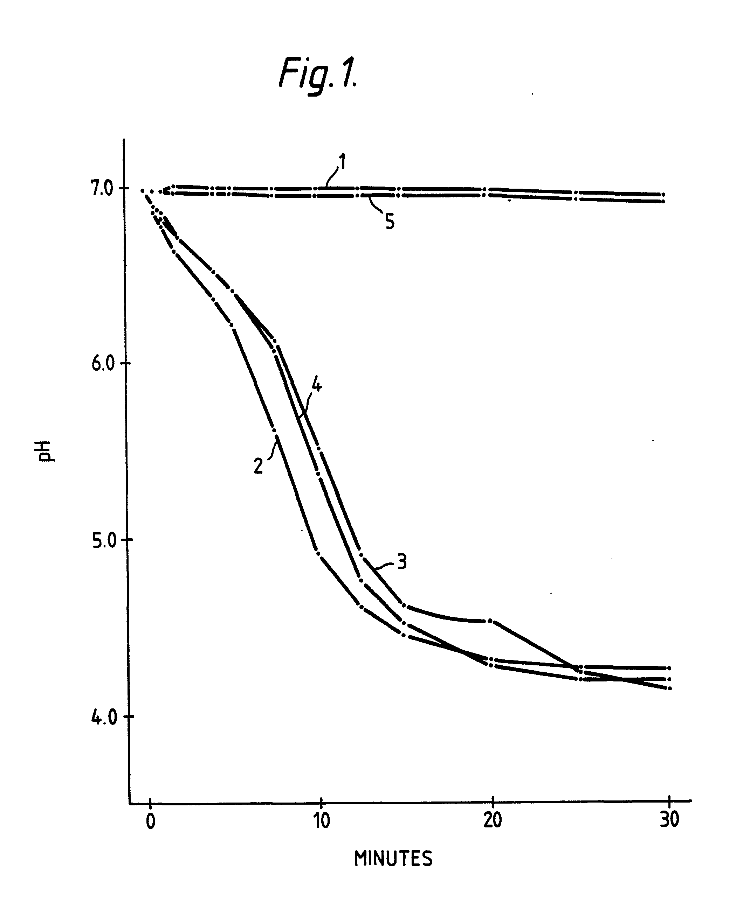

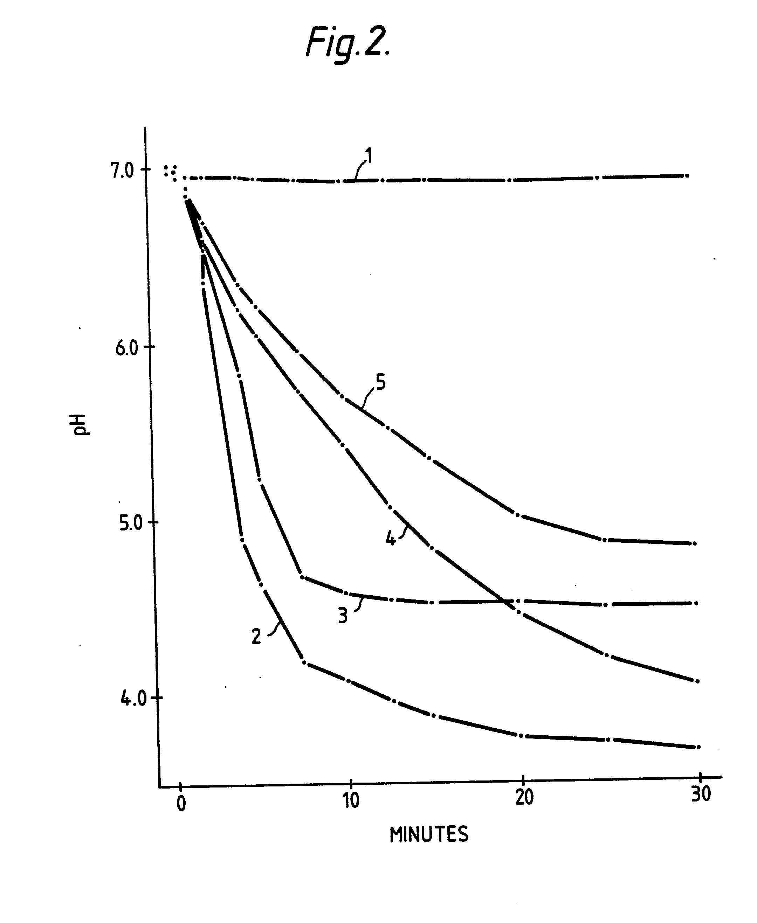

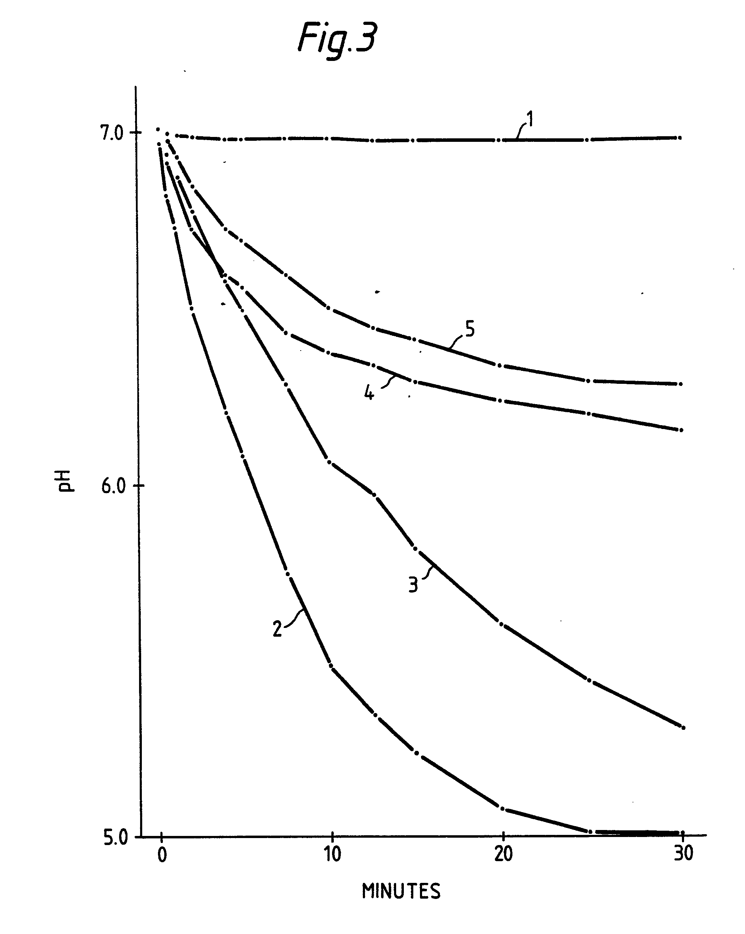

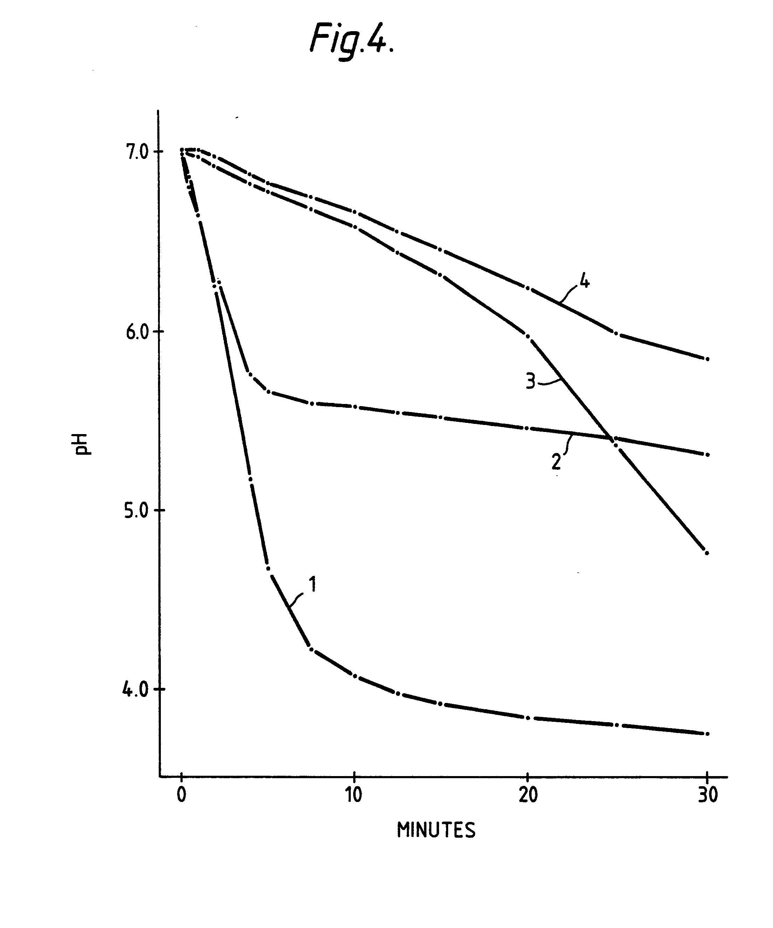

CLAIMS (for states other than Austria)

1. A composition for reducing the incidence
or severity of human dental caries, the composition
comprising fluoride ion and a nonmetabolizable
analogue of a cariogenic carbohydrate, in association
with a pharmaceutically acceptable carrier.

2. A composition as claimed in claim 1, wherein
the fluoride ion is that of sodium fluoride,
lithium fluoride, hydrogen fluoride, stannous fluoride,
rubidium fluoride, zirconium tetrafluoride or sodium
fluorophosphate.

3. A composition as claimed in claim 1 or 2,
wherein the cariogenic carbohydrate is glucose,
fructose, maltose, lactose or sucrose.

4. A composition as claimed in claims 2 and 3,
wherein the fluoride ion is that of sodium fluoride
and the cariogenic carbohydrate is sucrose.

5. A composition as claimed in claim 3 or 4, wherein
the nonmetabolizable analogue of a cariogenic
carbohydrate is 2-deoxy-D-glucose.

6. A composition as claimed in claim 3, wherein
the nonmetabolizable analogue of a cariogenic
carbohydrate is glucosamine or a pharmaceutically
acceptable salt thereof.

7. For use in reducing the incidence or severity
of human dental caries, fluoride ion and a
nonmetabolizable analogue of a cariogenic carbohydrate.

8. For use in reducing the incidence or severity
of human dental caries, a composition comprising
fluoride ion and a nonmetabolizable analogue of a
cariogenic carbohydrate in association with a
pharmaceutically acceptable carrier.

**0055109**

CLAIMS   (for Austria)

1. A method of preparing a composition for reducing the incidence or severity of human dental caries, which method comprises associating fluoride ion and a nonmetabolizable analogue of a cariogenic carbohydrate with a pharmaceutically acceptable carrier.

2. A method as claimed in claim 1, wherein the fluoride ion is that of sodium fluoride, lithium fluoride, hydrogen fluoride, stannous fluoride, rubidium fluoride, zirconium tetrafluoride or sodium fluorophosphate.

3. A method as claimed in claim 1 or 2, wherein the cariogenic carbohydrate is glucose, fructose, maltose, lactose or sucrose.

4. A method as claimed in claim 2 and 3, wherein the fluoride ion is that of sodium fluoride and the cariogenic carbohydrate is sucrose.

5. A method as claimed in claim 3 or 4, wherein the nonmetabolizable analogue of a cariogenic carbohydrate is 2-deoxy-D-glucose.

6. A method as claimed in claim 3, wherein the nonmetabolizable analogue of a cariogenic carbohydrate is glucosamine or a pharmaceutically acceptable salt thereof.

# Fig.1.

Fig.2.

Fig.3

*Fig.4.*